(19) Europäisches Patentamt
European Patent Office
Office européen des brevets

(11) Publication number: **0 008 507**
**B1**

# (12) EUROPEAN PATENT SPECIFICATION

(45) Date of publication of patent specification: **13.02.85**

(21) Application number: **79301562.9**

(22) Date of filing: **03.08.79**

(51) Int. Cl.[4]: **C 07 D 263/14**, C 08 K 5/35, C 08 L 23/12, C 08 L 23/16, C 09 K 15/30

## (54) Derivatives of 2-oxazolines as antioxidants and compositions thereof.

(30) Priority: **04.08.78 US 931087**
**30.05.79 US 43787**

(43) Date of publication of application:
**05.03.80 Bulletin 80/05**

(45) Publication of the grant of the patent:
**13.02.85 Bulletin 85/07**

(84) Designated Contracting States:
**AT BE CH DE FR GB IT NL**

(56) References cited:
**FR-A-2 032 828**
**FR-A-2 306 982**
**GB-A-1 217 941**

**Taschenbuch der Kunststoff-Additive, (Gächer und Müller), Carl Hanser Verlag, München Wien 1979 (pp. 7,67)**

**The file contains technical information submitted after the application was filed and not included in this specification**

(73) Proprietor: **UNIROYAL, INC.**
**1230 Avenue of the Americas Rockefeller Center**
**New York, New York 10020 (US)**

(72) Inventor: **Wheeler, Edward Lockwood**
**126 Claxton Avenue Watertown**
**Litchfield, Connecticut (US)**
Inventor: **Jancis, Elmar Harry**
**89 Spruce Drive Naugatuck**
**New Haven, Connecticut (US)**
Inventor: **Gencarelli, Richard Anthony**
**60 Maple Shade Road Middletown**
**Middlesex, Connecticut (US)**
Inventor: **Barrows, Franklin Herbert**
**20 Stoddard Place Beacon Falls**
**New Haven, Connecticut (US)**

(74) Representative: **Harrison, Michael Robert et al**
**URQUHART-DYKES & LORD 11th Floor Tower House Merrion Way**
**Leeds LS2 8PB West Yorkshire (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 008 507 B1

**Description**

The present invention relates to derivatives of 2-oxazolines useful as antioxidants.

The present invention relates to a substituted oxazoline characterised by having the structural formula

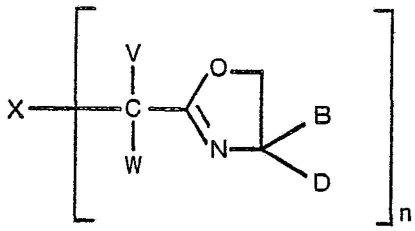

wherein B and D are the same or different and are methyl, ethyl, —$CH_2OH$ or $CH_2OOCY$, wherein Y is $C_1$—$C_{17}$ alkyl, phenyl or A; D may also be —$CH_2OOCZ$, wherein Z is a single bond connecting two groups of the said structural formula; A is 2-(3,5-di-t-butyl-4-hydroxyphenyl)ethyl; X is $C_1$—$C_{17}$ alkyl, phenyl, t-butylphenyl, nitrophenyl, hydroxyphenyl, chlorophenyl, (3,5-di-t-butyl-4-hydroxyphenyl)methyl or a divalent group selected from $C_1$—$C_2$ alkylene or thiodimethylene; V and W may be the same or different and are hydrogen, —$CH_2OH$ or —$CH_2OOCA$, wherein A has the meaning above; n is equal to 1 or 2; provided that X is not a divalent group when D is —$CH_2OOCZ$ and further provided that at least one of B, D, V, W and A contains a (3,5-di-t-butyl-4-hydroxyphenyl) moiety.

The compounds of the present invention are useful as antioxidants for protecting organic materials that are normally subject to oxidative degradation.

The present invention also provides a stabilised composition comprising an organic material normally subjected to deterioration by the influence of oxygen, light or heat characterised by said material containing from 0.001 to 10 parts per 100 parts of said material a substituted oxazoline of the invention.

The compounds of the present invention may be prepared by reacting a carboxylic acid, acid chloride or carboxylic ester with a beta-amino alcohol in a suitable solvent at 90—220°C, driving off two moles of water or one mole of alchol and one mole of water per mole of acid or ester to give the oxazoline. Those skilled in the art are aware of other methods of preparing oxazolines. Examples of suitable acids are formic, acetic, propionic, butyric, hexanoic, decanoic, lauric, stearic, cyclohexanecarboxylic, benzoic, 4-nitrobenzoic, 4-t-butylbenzoic, 2-chlorobenzoic, salicylic, phenylacetic, adipic, sebacic, azaleic, terephthalic, toluic, thiodipropionic and 3-(3,5-di-t-butyl-4-hydroxyphenyl) propionic. Examples of suitable esters are methyl formate, ethyl acetate, ethyl propionate, methyl laurate, methyl stearate, methyl benzoate, methyl salicylate, dimethyl adipate, dimethyl terephthalate, dimethyl thiodipropionate and methyl 3-(3,5-di-t-butyl-4-hydroxyphenyl) propionate. Examples of suitable 2-aminoalcohols are ethanolamine, 2-amino-1-propanol, 2-amino-2-methyl-1-propanol, 2-amino-2-methylpropanediol-1,3, 2-amino-ethylpropanediol-1,3, 2-amino-1-butanol and tris(hydroxymethyl)-aminomethane, l-isoleucinol. Examples of suitable sovlents are hexane, benzene, toluene, xylene, diethylbenzene, mesitylene, decalin, tetralin and other aliphatic or aromatic hydrocarbons.

When the 2-aminoalcohol contains two or more hydroxy groups, the resulting oxazoline has at least one unreacted hydroxy group. These hydroxy groups may be esterified by any of the methods known to those skilled in the art. Examples of suitable acids that may be used in such esterifications are formic, acetic, propionic, butyric, hexanoic, decanoic, lauric, stearic, cyclohexanecarboxylic, benzoic, 4-nitrobenzoic, 4-t-butylbenzoic, 2-chlorbenzoic, salicylic, phenylacetic, oxalic, succinic, maleic, malonic, adipic, sebacic, azelaic, phthalic, terephthalic, toluic, thiodipropionic and 3-(3,5-di-t-butyl-4-hydroxyphenyl)propionic. Examples of suitable acid chlorides that may be used in such esterifications are formoyl, acetyl, propionoyl, hexanoyl, dodecanoyl, stearoyl, benzoyl, 4-nitrobenzoyl, 2-chlorobenzoyl, oxalyl, succinoyl, adipoyl, sebacyl, phthaloyl, terephthaloyl, thiodipropionyl and 3-(3,5-di-t-butyl-4-hydroxyphenyl)propionyl. Examples of suitable anhydrides that may be used in such esterifications are succinic, maleic and phthalic. Examples of suitable esters that may be used for trans-esterification include methyl formate, methyl acetate, ethyl acetate, methyl propionate, methyl laurate, methyl stearate, methyl benzoate, methyl salicylate, diethyl oxalate, dimethyl adipate, dimethyl sebacate, dimethyl phthalate, dimethyl terephthalate, dimethyl thiodipropionate, and methyl 3-(3,5-di-t-butyl-4-hydroxyphenyl)-propionate. Examples of suitable solvents that may be used in esterifications and transesterifications are diethyl ether, dichloroethane, hexane, petroleum ether, benzene, toluene, xylene, dimethyl formamide, dimethyl sulfoxide, triethylamine, tributylamine, and pyridine.

If the group X of the compound of formula I possesses one or two hydrogens on the carbon alpha to the oxazoline ring, it may be hydroxymethylated with formaldehyde or formaldehyde donors in a suitable solvent. Suitable formaldehyde compositions include formaldehyde (gas), aqueous formaldehyde, paraformaldehyde and methyl formcel. Examples of suitable solvents are hexane, benzene, toluene, xylene and diethyl benzene. The alpha-hydroxymethyl derivatives thus prepared may then be esterified by those methods described above.

Non-limiting examples of compounds of the invention are the following:

2-(4,4-Dimethyl-2-oxazoline)-2-methyltrimethylene bis[3-(3,5-di-t-butyl-4-hydroxyphenyl)propionate]
2-(4,4-Dimethyl-2-oxazoline)-2-butyltrimethylene bis[3-(3,5-di-t-butyl-4-hydroxyphenyl)propionate]
2-(4,4-Dimethyl-2-oxazoline)-2-decyltrimethylene bis[3-(3,5-di-t-butyl-4-hydroxyphenyl)propionate]
2-(4,4-Dimethyl-2-oxazoline)-2-hexadecyltrimethylene bis[3-(3,5-di-t-butyl-4-hydroxyphenyl)propionate]
2-(4,4-Dimethyl-2-oxazoline)-2-phenyltrimethylene bis[3-(3,5-di-t-butyl-4-hydroxyphenyl)propionate]
2-(4,4-Dimethyl-2-oxazoline)-2-methylpropyl 3-(3,5-di-t-butyl-4-hydroxyphenyl)propionate
2-(4,4-Dimethyl-2-oxazoline)-2-methylstearyl 3-(3,5-di-t-butyl-4-hydroxyphenyl)propionate
2-(4,4-Dimethyl-2-oxazoline)isobutanetriyl tris[3-(3,5-di-t-butyl-4-hydroxyphenyl)propionate]
2-Methyl-2-(4-methyl-2-oxazoline)trimethylene bis[3-(3,5-di-t-butyl-4-hydroxyphenyl)propionate]
2-Butyl-2-(4-methyl-2-oxazoline)trimethylene bis[3-(3,5-di-t-butyl-4-hydroxyphenyl)propionate]
2-Decyl-2-(4-methyl-2-oxazoline)trimethylene bis[3-(3,5-di-t-butyl-4-hydroxyphenyl)propionate]
2-Hexadecyl-2-(4-methyl-2-oxazoline)trimethylene bis[3-(3,5-di-t-butyl-4-hydroxyphenyl)propionate]
2-Phenyl-2-(4-methyl-2-oxazoline)trimethylene bis[3-(3,5-di-t-butyl-4-hydroxyphenyl)propionate]
2-Methyl-2-(4-methyl-2-oxazoline)propyl 3-(3,5-di-t-butyl-4-hydroxyphenyl)propionate
2-Methyl-2-(4-methyl-2-oxazoline)stearyl 3-(3,5-di-t-butyl-4-hydroxyphenyl)propionate
2-(4-Methyl-2-oxazoline)isobutanetriyl tris[3-(3,5-di-t-butyl-4-hydroxyphenyl)propionate]
2-Methyl-2-[4-(1-methylpropyl)-2-oxazoline]trimethylene bis[3-(3,5-di-t-butyl-4-hydroxyphenyl)propionate]
2-Butyl-2-[4-(1-methylpropyl)-2-oxazoline]trimethylene bis[3-(3,5-di-t-butyl-4-hydroxyphenyl)propionate]
2-Decyl-2-[4-(1-methylpropyl)-2-oxazoline]trimethylene bis[3-(3,5-di-t-butyl-4-hydroxyphenyl)propionate]
2-Hexadecyl-2-[4-(1-methylpropyl)-2-oxazoline]trimethylene bis[3-(3,5-di-t-butyl-4-hydroxyphenyl)-propionate]
2-Phenyl-2-[4-(1-methylpropyl)-2-oxazoline]trimethylene bis[3-(3,5-di-t-butyl-4-hydroxyphenyl)propionate]
2-Methyl-2-[4-(1-methylpropyl)-2-oxazoline]propyl 3-(3,5-di-t-butyl-4-hydroxyphenyl)propionate]
2-Methyl-2-[4-(1-methylpropyl)-2-oxyazoline]stearyl 3-(3,5-di-t-butyl-4-hydroxyphenyl)propionate
2-[4-(1-Methylpropyl)-2-oxazoline]isobutanetriyl tris[3-(3,5-di-t-butyl-4-hydroxyphenyl)propionate]
2-(3,5-Di-t-butyl-4-hydroxybenzyl)-2-(4,4-dimethyl-2-oxazoline)trimethylene diacetate
2-(3,5-Di-t-butyl-4-hydroxybenzyl)-2-(4,4-dimethyl-2-oxazoline)trimethylene dihexanoate
2-(3,5-Di-t-butyl-4-hydroxybenzyl)-2-(4,4-dimethyl-2-oxazoline)trimethylene didodecanoate
2-(3,5-Di-t-butyl-4-hydroxybenzyl)-2-(4,4-dimethyl-2-oxazoline)trimethylene distearate
2-(3,5-Di-t-butyl-4-hydroxybenzyl)-2-(4,4-dimethyl-2-oxazoline)trimethylene dibenzoate
2-(3,5-Di-t-butyl-4-hydroxybenzyl)-2-(4,4-dimethyl-2-oxazoline)trimethylene ditoluate
2-(3,5-Di-t-butyl-4-hydroxybenzyl)-2-(4,4-dimethyl-2-oxazoline)trimethylene ditoluate
2-(3,5-Di-t-butyl-4-hydroxybenzyl)-2-(4,4-dimethyl-2-oxazoline)trimethylene di(butylbenzoate)
2-(3,5-Di-t-butyl-4-hydroxybenzyl)-2-(4,4-dimethyl-2-oxazoline)propanol
2-(3,5-Di-t-butyl-4-hydroxybenzyl)-2-(4,4-dimethyl-2-oxazoline)propyl 3-(3,5-di-t-butyl-4-hydroxyphenyl)-propionate
2-(3,5-Di-t-butyl-4-hydroxybenzyl)-2-(4,4-dimethyl-2-oxazoline)propyl acetate
2-(3,5-Di-t-butyl-4-hydroxybenzyl)-2-(4,4-dimethyl-2-oxazoline)propyl hexanoate
2-(3,5-Di-t-butyl-4-hydroxybenzyl)-2-(4,4-dimethyl-2-oxazoline)propyl dodecanoate
2-(3,5-Di-t-butyl-4-hydroxybenzyl)-2-(4,4-dimethyl-2-oxazoline)propyl stearate
2-(3,5-Di-t-butyl-4-hydroxybenzyl)-2-(4,4-dimethyl-2-oxazoline)propyl benzoate
2-(3,5-Di-t-butyl-4-hydroxybenzyl)-2-(4,4-dimethyl-2-oxazoline)propyl toluate
2-(3,5-Di-t-butyl-4-hydroxybenzyl)-2-(4-methyl-2-oxazoline)propanediol-1,3
2-(3,5-Di-t-butyl-4-hydroxybenzyl)-2-(4-methyl-2-oxazoline)trimethylene bis[3-(3,5-di-t-butyl-4-hydroxyphenyl)propionate]
2-(3,5-Di-t-butyl-4-hydroxybenzyl)-2-(4-methyl-2-oxazoline)trimethylene diacetate
2-(3,5-Di-t-butyl-4-hydroxybenzyl)-2-(4-methyl-2-oxazoline)trimethylene dihexanoate
2-(3,5-Di-t-butyl-4-hydroxybenzyl)-2-(4-methyl-2-oxazoline)trimethylene didodecanoate
2-(3,5-Di-t-butyl-4-hydroxybenzyl)-2-(4-methyl-2-oxazoline)trimethylene distearate
2-(3,5-Di-t-butyl-4-hydroxybenzyl)-2-(4-methyl-2-oxazoline)trimethylene dibenzoate
2-(3,5-Di-t-butyl-4-hydroxybenzyl)-2-(4-methyl-2-oxazoline)trimethylene ditoluate
2-(3,5-Di-t-butyl-4-hydroxybenzyl)-2-[4-(1-methylpropyl)-2-oxazoline] propandiol-1,3
2-(3,5-Di-t-butyl-4-hydroxybenzyl)-2-[4-(1-methylpropyl)-2-oxazoline]trimethylene bis[3-(3,5-di-t-butyl-4-hydroxyphenyl)-propionate]
2-(3,5-Di-t-butyl-4-hydroxybenzyl)-2-[4-(1-methylpropyl)-2-oxazoline]-trimethylene diacetate
2-(3,5-Di-t-butyl-4-hydroxybenzyl)-2-[4-(1-methylpropyl)-2-oxazoline]-trimethylene dihexanoate
2-(3,5-Di-t-butyl-4-hydroxybenzyl)-2-[4-(1-methylpropyl)-2-oxazoline]-trimethyl didodecanoate
2-(3,5-Di-t-butyl-4-hydroxybenzyl)-2-[4-(1-methylpropyl)-2-oxazoline]-trimethylene distearate
2-(3,5-Di-t-butyl-4-hydroxybenzyl)-2-[4-(1-methylpropyl)-2-oxazoline]-trimethylene dibenzoate
2-(3,5-Di-t-butyl-4-hydroxybenzyl)-2-[4-(1-methylpropyl)-2-oxazoline]-trimethylene ditoluate
1-(3,5-Di-t-butyl-4-hydroxybenzyl)-1-(4,4-dimethyl-2-oxazoline) ethene
1-(3,5-Di-t-butyl-4-hydroxybenzyl)-1-(4,-methyl-2-oxazoline) ethene
1-(3,5-Di-t-butyl-4-hydroxybenzyl)-1-[4,-(1-methylpropyl)-2-oxazoline]ethene

2-[1-(3,5-Di-t-butyl-4-hydroxybenzyl)ethenyl]-2-oxazoline-4,4-dimethyl bis[3-(3,5-di-t-butyl-4-hydroxyphenyl)-propionate]
2-(1-methylethenyl)-2-oxazoline-4,4-dimethyl bis[3-(3,5-di-t-butyl-4-hydroxyphenyl)propionate]
2-(1-Butylethenyl)-2-oxazoline-4,4-dimethyl bis[3-(3,5-di-t-butyl-4-hydroxyphenyl)propionate]
2-(1-Decylethenyl)-2-oxazoline-4,4-dimethyl bis[3-(3,5-di-t-butyl-4-hydroxyphenyl)propionate]
2-(1-Hexadecylethenyl)-2-oxazoline-4,4-dimethyl bis[3-(3,5-di-t-butyl-4-hydroxyphenyl)propionate]
2-(1-phenylethenyl)-2-oxazoline-4,4-dimethyl bis[3-(3,5-di-t-butyl-4-hydroxyphenyl)propionate]
2-[1-(3,5-Di-t-butyl-4-hydroxybenzyl)ethenyl]-4-methyl-2-oxazoline-4-methyl [3-(3,5-di-t-butyl-4-hydroxyphenyl)propionate]
2-[1-(3,5-Di-t-butyl-4-hydroxybenzyl)ethenyl]-4-ethyl-2-oxazoline-4-methyl 3-(3,5-di-t-butyl-4-hydroxyphenyl)-propionate
2-(1-Methylethenyl)-4-methyl-2-oxazoline-4-methyl 3-(3,5-di-t-butyl-4-hydroxyphenyl)propionate
2-(1-Butylethenyl)-4-methyl-2-oxazoline-4-methyl 3-(3,5-di-t-butyl-4-hydroxyphenyl)propionate
2-(1-Decylethenyl)-4-methyl-2-oxazoline-4-methyl 3-(3,5-di-t-butyl-4-hydroxyphenyl)propionate
2-(1-Hexadecylethenyl)-4-methyl-2-oxazoline-4-methyl 3-(3,5-di-t-butyl-4-hydroxyphenyl)propionate
2-(1-Phenylethenyl)-4-methyl-2-oxazoline-4-methyl 3-(3,5-di-t-butyl-4-hydroxyphenyl)propionate
2-(1-Methylethenyl)-4-ethyl-2-oxazoline-4-methyl 3-(3,5-di-t-butyl-4-hydroxyphenyl)propionate
2-(1-Butylethenyl)-4-ethyl-2-oxazoline-4-methyl 3-(3,5-di-t-butyl-4-hydroxyphenyl)propionate
2-(1-Decylethenyl)-4-ethyl-2-oxazoline-4-methyl 3-(3,5-di-t-butyl-4-hydroxyphenyl)propionate
2-(1-Hexadecylethenyl)-4-ethyl-2-oxazoline-4-methyl 3-(3,5-di-t-butyl-4-hydroxyphenyl)propionate
2-(1-Phenylethenyl)-4-ethyl-2-oxazoline-4-methyl 3-(3,5-di-t-butyl-4-hydroxyphenyl)propionate
2-[2-(3,5-Di-t-butyl-4-hydroxyphenyl)-1,1-bis(hydroxymethyl)ethyl]-4-hydroxymethyl-4-methyl-2-oxazoline
2-[2-(3,5-Di-t-butyl-4-hydroxyphenyl)-1,1-bis(hydroxymethyl)ethyl]-4-methyl-2-oxazoline-4-methylene 3-(3,5-di-t-butyl-4-hydroxyphenyl)propionate
2-[2-(3,5-Di-t-butyl-4-hydroxyphenyl)-1,1-bis(hydroxymethyl]ethyl)-4-ethyl-2-oxazoline-4-methyl 3-(3,5-di-t-butyl-4-hydroxyphenyl)propionate
2-(3,5-Di-t-butyl-4-hydroxybenzyl)-2-[4-methyl-2-oxazoline-4-[2-(3,5-di-t-butyl-4-hydroxyphenyl)propanoyl-oxymethyl]]trimethylene bis[3-(3,5-di-t-butyl-4-hydroxyphenyl)propionate]
2-(3,5-Di-t-butyl-4-hydroxybenzyl)-2-[4-ethyl-2-oxazoline-4-[2-(3,5-di-t-butyl-4-hydroxyphenyl)propanoyl-oxymethyl]]trimethylene bis[3-(3,5-di-t-butyl-4-hydroxyphenyl)propionate]
2-Methyl-2-[4-methyl-2-oxazoline-4-[2-(3,5-di-t-butyl-4-hydroxyphenyl)propanoyloxymethyl]]trimethylene bis[3-(3,5-di-t-butyl-4-hydroxyphenyl)propionate]
2-Butyl-2-[4-methyl-2-oxazoline-4-[2-(3,5-di-t-butyl-4-hydroxyphenyl)propanoyloxymethyl]]trimethylene bis[3-(3,5-di-t-butyl-4-hydroxyphenyl)propionate]
2-Decyl-2-[4-methyl-2-oxazoline-4-[2-(3,5-di-t-butyl-4-hydroxyphenyl)propanoyloxymethyl]]trimethylene bis[3-(3,5-di-t-butyl-4-hydroxyphenyl)propionate].
2-Hexadecyl-2-[4-methyl-2-oxazoline-4-[2-(3,5-di-t-butyl-4-hydroxyphenyl)propanoyloxymethyl]]-trimethylene bis[3-(3,5-di-t-butyl-4-hydroxyphenyl)propionate]
2-Phenyl-2-[4-methyl-2-oxazoline-4-[2-(3,5-di-t-butyl-4-hydroxyphenyl)propanoyloxymethyl]]trimethylene bis[3-(3,5-di-t-butyl-4-hydroxyphenyl)propionate]
2-Methyl-2-[4-ethyl-2-oxazoline-4-[2-(3,5-di-t-butyl-4-hydroxyphenyl)propanoyloxymethyl]]trimethylene bis[3-(3,5-di-t-butyl-4-hydroxyphenyl)propionate]
2-Butyl-2-[4-ethyl-2-oxazoline-4-[2-(3,5-di-t-butyl-4-hydroxyphenyl)propanoyloxymethyl]]trimethylene bis[3-(3,5-di-t-butyl-4-hydroxyphenyl)propionate]
2-Decyl-2-[4-ethyl-2-oxazoline-4-[2-(3,5-di-t-butyl-4-hydroxyphenyl)propanoyloxymethyl]]trimethylene bis[3-(3,5-di-t-butyl-4-hydroxyphenyl)propionate]
2-Hexadecyl-2-[4-ethyl-2-oxazoline-4-[2-(3,5-di-t-butyl-4-hydroxyphenyl)propanoyloxymethyl]]-trimethylene bis[3-(3,5-di-t-butyl-4-hydroxyphenyl)propionate]
2-Phenyl-2-[4-ethyl-2-oxazoline-4-[2-(3,5-di-t-butyl-4-hydroxyphenyl)propanoyloxymethyl]]trimethylene bis[3-(3,5-di-t-butyl-4-hydroxyphenyl)propionate]
2-[2-(3,5-Di-t-butyl-4-hydroxyphenyl)-1,1-bis(hydroxymethyl)ethyl]-2-oxazoline-4,4-dimethyl bis[3-(3,5-di-t-butyl-4-hydroxyphenyl)propionate]
2-[2-(3,5-Di-t-butyl-4-hydroxyphenyl)-1,1-bis(ethanoyloiylmethyl)ethyl]-2-oxazoline-4,4-dimethyl bis[3-(3,5-di-t-butyl-4-hydroxyphenyl)propionate]
2-[2-(3,5-Di-t-butyl-4-hydroxyphenyl)-1,1-bis(hexanoyloxymethyl)ethyl]-2-oxazoline-4,4-dimethyl bis[3-(3,5-di-t-butyl-4-hydroxyphenyl)propionate]
2-[2-(3,5-Di-t-butyl-4-hydroxyphenyl)-1,1-bis(dodecanoyloxymethyl)ethyl]-2-oxazoline-4,4-dimethyl bis[3-(3,5-di-t-butyl-4-hydroxyphenyl)propionate]
2-[2-(3,5-Di-t-butyl-4-hydroxyphenyl)-1,1-bis(octadecanoyloxymethyl)ethyl]-2-oxazoline-4,4-dimethyl bis[3-(3,5-di-t-butyl-4-hydroxyphenyl)propionate]
2-[2-(3,5-Di-t-butyl-4-hydroxyphenyl)-1,1-bis(benzenoyloxymethyl)ethyl]-2-oxazoline-4,4-dimethyl bis[3-(3,5-di-t-butyl-4-hydroxyphenyl)propionate]
2-[2-(3,5-Di-t-butyl-4-hydroxyphenyl)-1,1-bis(ethanoyloxymethyl)ethyl]-4-methyl-2-oxazoline-4-methyl 3-(3,5-di-t-butyl-4-hydroxyphenyl)propionate

2-[2-(3,5-Di-t-butyl-4-hydroxyphenyl)-1,1-bis(hexanoyloxymethyl)ethyl]-4-methyl-2-oxazoline-4-methyl 3-(3,5-di-t-butyl-4-hydroxyphenyl)propionate

2-[2-(3,5-Di-t-butyl-4-hydroxyphenyl)-1,1-bis(dodecanoyloxymethyl)ethyl]-4-methyl-2-oxazoline-4-methyl 3-(3,5-di-t-butyl-4-hydroxyphenyl)propionate

2-[2-(3,5-Di-t-butyl-4-hydroxyphenyl)-1,1-bis(octadecanoyloxymethyl)ethyl]-4-methyl-2-oxazoline-4-methyl 3-(3,5-di-t-butyl-4-hydroxyphenyl)propionate

2-[2-(3,5-Di-t-butyl-4-hydroxyphenyl)-1,1-bis(benzenoyloxymethyl)ethyl]-4-methyl-2-oxazoline-4-methyl 3-(3,5-di-t-butyl-4-hydroxyphenyl)propionate

2-[2-(3,5-Di-t-butyl-4-hydroxyphenyl)-1,1-bis(ethanoyloxymethyl)ethyl]-4-ethyl-2-oxazoline-4-methyl 3-(3,5-di-t-butyl-4-hydroxyphenyl)propionate

2-[2-(3,5-Di-t-butyl-4-hydroxyphenyl)-1,1-bis(hexanoyloxymethyl)ethyl]-4-ethyl-2-oxazoline-4-methyl 3-(3,5-di-t-butyl-4-hydroxyphenyl)propionate

2-[2-(3,5-Di-t-butyl-4-hydroxyphenyl)-1,1-bis(dodecanoyloxymethyl)ethyl]-4-ethyl-2-oxazoline-4-methyl 3-(3,5-di-t-butyl-4-hydroxyphenyl)propionate

2-[2-(3,5-Di-t-butyl-4-hydroxyphenyl)-1,1-bis(octadecanoyloxymethyl)ethyl]-4-ethyl-2-oxazoline-4-methyl 3-(3,5-di-t-butyl-4-hydroxyphenyl)propionate

2-[2-(3,5-Di-t-butyl-4-hydroxyphenyl)-1,1-bis(benzenoyloxymethyl)ethyl]-4-ethyl-2-oxazoline-4-methyl 3-(3,5-di-t-butyl-4-hydroxyphenyl)propionate

The compounds of the present invention are useful in stabilizing organic material normally subject to oxidative degradation. Materials that are thus stabilized include a multitude of synthetic polymers. Among such polymers are various polyolefins such as polyethylene, polypropylene, polybutylene, polybutadiene and polymethylpentene. Other polymers include acetal resins, polyacrylates, polymethacrylates, polydialkylphthalate, cellulosics, polyamides, polyesters, polyurethanes, polycarbonate, polystyrene, polyvinyl chloride and polyvinylidene chloride. Copolymers can also be stabilized by the compounds of the present invention. Representative copolymers include ethylene/propylene copolymers, butadiene/styrene copolymers, ethylene-vinyl acetate copolymers, and ethylene/ethyl acrylate copolymers. Copolymers also includes terpolymers such as ethylene propylene terpolymers (these terpolymers are preferentially ethylene, propylene, non-conjugated diene copolymers) and acrylonitrile/butadiene/styrene copolymers. Polymer blends such as polystyrene/polyphenylene oxide and ethylenepropylene copolymer/polypropylene can also be stabilized by the compounds of the present invention. Other materials stabilized by the compounds of the present invention include hot melt adhesives such as those based on polyesters, polyamides or ethylene/vinyl acetate. Also stabilized are petroleum products such as fuels, lubricating oils and petrolatum jellies, and natural products such as natural rubber, waxes, fat, tallow, linseed oil, corn oil, cotton seed oil and codliver oil.

The preceding list is representative, though by no means exhaustive, of the products that can benefit from the compounds of the present invention. To achieve protection against oxidative degradation, the compounds of the present invention are generally added in the same amounts used for other antioxidants. Generally, depending on the substrate used the antioxzidant is added in amounts of 0.001 to 10% by weight, based on the weight of the substrate, with the more usual range being 0.05 to 2.0%. The compounds of the present invention can be used by themselves to stabilize organic materials, or they can be used in combination with other stabilizers. Such other stabilizers might include other phenolics, thio compounds of various kinds, such as thiodipropionate esters, phosphites and phosphonates, anti-copper chemicals such as oxalamides, and ultraviolet stabilizers of various kinds as well as other additives where the use of such additives has been found to be beneficial.

### Example 1

2-[2-(3,5-Di-t-butyl-4-hydroxyphenyl)ethyl]-4,4-dimethyl-2-oxazoline

In a 1 liter, three-neck, round-bottom flask equipped with a thermometer, stirrer and a Stark and Dean trap with condenser was placed 139 g (0.5 moles) of 3-(3,5-di-t-butyl-4-hydroxyphenyl)propionic acid, 400 ml of xylene and 44.5 g (0.5 moles) of 2-amino-2-methyl-1-propanol (AMP). The mixture was refluxed for 20 hours and 20 ml of water were collected. The title compound crystallized from the cooled mixture and upon drying melted at 139—141°C.

### Example 2

4-Hydroxymethyl-4-methyl-2-[2-(3,5-di-t-butyl-4-hydroxyphenyl)-ethyl]-2-oxazoline

Prepared as described in Example 1 except that 0.5 mole of 2-amino-methylpropanediol-1,3 was used instead of aminomethylpropanol. Recrystallization from cyclohexane gave a compound that melts at 116—119°C.

### Example 3

4-Hydroxymethyl-4-ethyl-2-[2-(3,5-di-ti-butyl-4-hydroxyphenyl)-ethyl] oxazoline

Prepared as described in Example 1 except that 0.5 mole of 2-amino-2-ethylpropanediol-1,3 was used instead of aminomethylpropanol. Recrystallization from cyclohexane gave a compound that melts at 123—125°C.

Example 4

2-[2-(3,5-Di-t-butyl-4-hydroxyphenyl)ethyl]-4-methyl 2-oxazoline-4-methyl[3-(3,5-di-t-butyl-4-hydroxyphenyl)propionate]

Prepared as described in Example 1 except that 0.25 mole of 2-amino-2-methylpropanediol-1,3 was used instead of 0.5 mole aminomethylpropanol. The compounds melted at 163—165°C.

Example 5

2-[2-(3,5-di-t-butyl-4-hydroxyphenyl)ethyl]-4-ethyl-2-oxazoline-4-methyl[3-(3,5-di-t-butyl-4-hydroxyphenyl)propionate]

Prepared as described in Example 1 except that 0.25 mole of 2-amino-2-ethylpropanediol-1,3 was used instead of 0.5 mole of aminomethyl propanol. Recrystallization from benzene gave a compound that melted at 140—141°C and had the following analysis:

| | C | H | N |
|---|---|---|---|
| Calculated | 75.32 | 9.56 | 2.25 |
| Found | 75.20 | 9.50 | 1.96 |

Example 6

2-[2-(3,5-Di-t-butyl-4-hydroxyphenyl)ethyl]-4,4-bis(hydroxymethyl)-2-oxazoline

Prepared as described in Example 1 except that 0.5 mole of tris(hydroxymethyl)amino methane (TRIS) was used instead of AMP. Slurrying with hot toluene gave a pure compound that melted at 175—176.5°C and had the following analysis:

| | C | H | N |
|---|---|---|---|
| Calculated | 68.34 | 9.46 | 3.90 |
| Found | 68.34 | 9.30 | 3.82 |

Example 7

2-[2-(3,5-Di-t-butyl-4-hydroxyphenyl)ethyl]-4-hydroxymethyl-2-oxazoline-4-methyl[3-(3,5-di-t-butyl-4-hydroxyphenyl)propionate]

In a 1 liter, three-neck, round-bottom flask equipped with a thermometer, stirrer and Stark and Dean trap with a condenser was placed 278 g (1.0 mole) of 3-(3,5-di-t-butyl-4-hydroxyphenyl)propionic acid, 60.5 g (0.5 mole) of tris(hydromethyl)aminomethane and 400 ml of xylene. The mixture was refluxed at 140°C for 8 hours and then heated at 190—195°C for 6 hours. A total of 27 ml of water was collected in the Stark and Dean trap. The mixture was diluted to 500 ml with xylene and allowed to cool. A solid separated and it was removed by filtration and dried. This solid proved to be a mixture of 2-[3-(3,5-di-t-butyl-4-hydroxyphenyl)propionamido]-2-bis(hydroxymethyl)-ethyl 3-(3,5-di-t-butyl-4-hydroxyphenyl)propionate that melted at 167—170°C and 2-[2-(3,5-di-t-butyl-4-hydroxyphenyl)ethyl]-2-oxazoline-4,4-dimethyl bis[3-(3,5-di-t-butyl-4-hydroxyphenyl)propionate] that melted at 173—175°C. The xylene solution was stripped and the residue dissolved in hot hexane. The title compound crystallized and it melted at 126—128°C.

Example 8

2-[2-(3,5-Di-t-butyl-4-hydroxyphenyl)ethyl]-2-oxazoline-4,4-dimethyl bis[3-(3,5-di-t-butyl-4-hydroxyphenyl)propionate]

In a 1 liter, three-neck, round-bottom flask equipped with a thermometer, stirrer and a Stark and Dean trap with a condenser was placed 167 g (0.6 mole) of 3-(3,5-di-t-butyl-4-hydroxyphenyl)propionic acid, 24.2 g (0.2 mole) of tris(hydroxymethyl)aminomethane and 100 ml of xylene. The mixture was heated to 172°C by removing xylene for 5.5 hours and 12.8 ml of water was collected. The reaction mixture was slurried with hot hexane and allowed to cool. The product melted at 158—160°C. This solid was recrystallized from benzene and gave a product that melted at 173—175°C and had the following analysis:

| | C | H | N |
|---|---|---|---|
| Calculated | 74.70 | 9.23 | 1.58 |
| Found | 74.53 | 9.09 | 1.34 |

Example 9

2-[2-(3,5-Di-t-butyl-4-hydroxyphenyl)-1,1-bis(hydroxymethyl)ethyl]-4,4-dimethyl-2-oxazoline

In a 1 liter, three-neck, round-bottom flask equipped with a thermometer, stirrer and condenser was placed 331 g (1.0 mole) of 2-[2-(3,5-di-t-butyl-4-hydroxyphenyl)ethyl]-4,4-dimethyl-2-oxazoline, 100 g of 91% paraformaldehyde and 1000 ml of xylene. The mixture was heated to 110—120°C for 3 hours. The mixture was filtered hot and allowed to cool. The product was removed by filtration and dried. The melting point was 123—125°C.

## Example 10

2-(3,5-Di-t-butyl-4-hydroxybenzyl)-2-(4,4-dimethyl-2-oxazoline)-trimethylene bis[3-(3,5-di-t-butyl-4-hydroxyphenyl)propionate]

In a 3 liter, three-neck, round-bottom flask equipped with a dropping funnel, stirrer and condenser was placed 195.5 g (0.5 mole) of 2-[2-(3,5-di-t-butyl-4-hydroxyphenyl)-1,1-bis(hydroxymethyl)ethyl]-4,4-dimethyl-2-oxazoline, 800 ml of hexane and 150 ml of triethylamine. To this stirred slurry at reflux was added 278 g of 3-(3,5-di-t-butyl-4-hydroxyphenyl)propionyl chloride in hexane. The mixture was filtered hot and the solid was washed with water. The product was not soluble in hexane or in water and melted at 205—215°C. This solid was slurried with hot methanol to give a pure product melting at 214—216°C.

## Example 11

4,4-Bis(hydroxymethyl)-2-methyl-2-oxazoline

In a 2 liter, three-neck, round-bottom flask equipped with a thermometer, stirrer and Stark and Dean trap with a condenser was placed 363 g (3.0 mole) of tris(hydroxymethyl)aminomethane, 400 ml of xylene and 180 g (3.0 mole) of acetic acid. The mixture was refluxed for 8 hours and 115 ml of water were collected. The mixture was allowed to cool and a solid was isolated. The solid was distilled at 1.5 mm and 148—155°C to give the produce which melted at 89—92°C. Two recrystallizations from ethyl acetate raised the melting point to 90—93°C.

## Example 12

4,4-Bis(hydroxymethyl)-2-n-pentyl-2-oxazoline

In a 2 liter, three-neck, round-bottom flask equipped with a thermometer, stirrer and a Stark and Dean trap with a condenser was placed 121 g (1.0 mole) of tris(hydroxymethyl)aminomethane, 116 g (1.0 mole) of n-hexanoic acid and 400 ml of xylene. The mixture was refluxed for 4 hours and then allowed to cool. The reaction mixture was diluted with 500 ml of hexane and the product was removed by filtration. The product melted at 76—78°C.

## Example 13

4,4-Bis(hydroxymethyl)-2-n-undecyl-2-oxazoline

Prepared as described in Example 12 except that 1.0 mole of dodecanoic acid was used instead of hexanoic acid. The melting point was 95—96°C.

## Example 14

4,4-Bis(hydroxymethyl)-2-n-heptadecyl-2-oxazoline

Prepared as described in Example 12 except that 1.0 mole of stearic acid was used instead of hexanoic acid. The product melted at 97—99°C.

## Example 15

2,2′-Tetramethylenebis [4,4-bis(hydroxymethyl)-2-oxazoline]

Prepared as described in Example 12 except that 0.5 mole of adipic acid was used instead of hexanoic acid. The product melted at 190—192°C.

## Example 16

2,2′-Thiodiethylenebis[4,4-bis(hydroxymethyl)-2-oxazoline]

Prepared as described in Example 12 except that 0.5 mole of 3,3′-thiodipropionic acid was used instead of hexanoic acid. The product melted at 136—138°C.

## Example 17

4,4-Bis(hydroxymethyl)-2-phenyl-2-oxazoline

In a 2 liter, three-neck, round-bottom flask equipped with a thermometer, stirrer and a Stark and Dean trap with condenser was placed 122 g (1.0 mole) of benzoic acid, 121 g (1.0 mole) of tris(hydroxymethyl)aminomethane and 400 ml of xylene. The mixture was heated to 140°C but no water distilled. The xylene was distilled off and the reaction temperature raised to 195°C. At this temperature the reaction was complete in 3 hours. The mixture was cooled and 100 ml of xylene and 500 ml of hexane were added. The product crystallized and was removed by filtration. One washing with acetone gave the product which melted at 138—140°C.

## Example 18

2,(4-t-Butylphenyl)-4,4-bis(hydroxymethyl)-2-oxazoline

Prepared as described in Example 17 except that 1.0 mole of 4-t-butylbenzoic acid was used instead of benzoic acid. The product melted at 138—140°C.

Example 19

4,4-Bis(hydroxymethyl)-2-(4-nitrophenyl)-2-oxazoline

Prepared as described in Example 17 except that 1.0 mole of 4-nitrobenzoic acid was used instead of benzoic acid. The product was recrystallized from methanol and melted at 194—196°C.

Example 20

2-(2-Chlorophenyl)-4,4-bis(hydroxymethyl)-2-oxazoline

Prepared as described in Example 17 except that 2-chlorobenzoic acid was used instead of benzoic acid. The product was recrystallized from a 25/75 by volume mixture of isopropanol-ethyl acetate and melted at 135—137°C.

Example 21

4,4-Bis(hydroxymethyl)-2-(2-hydroxyphenyl)-2-oxazoline

Prepared as described in Example 17 except that salicylic acid was used instead of benzoic acid. The product was recrystallized from ethylacetate and melted at 104—106°C.

Example 22

2-Methyl-2-oxazoline-4,4-dimethyl bis[3-(3,5-di-t-butyl-4-hydroxyphenyl)propionate]

In a 500 ml, three-neck, round-bottom flask equipped with a thermometer, stirrer and a Stark and Dean trap with condenser was placed 14.5 g (0.1 mole) of 4,4-bis(hydroxymethyl)-2-methyl-2-oxazoline, 55.6 g (0.2 mole) of 3-(3,5-di-t-butyl-4-hydroxyphenyl) propionic acid and 100 ml of xylene. The mixture was heated to reflux and 1.6 ml of water was collected in 3 hours. The evolution ceased so the temperature was raised to 185°C, by removing the xylene, and the remainder of the water collected. The reaction mixture was dissolved in hot hexane and allowed to cool. A solid crystallized which was removed by filtration and dried, melting at 121—124°C. This compound was identified as 2-[2-(3,5-di-t-butyl-4-hydroxyphenyl)ethyl]-4-[(acetoxy)-methyl]-2-oxazoline-4-methyl [3-(3,5-di-t-butyl-4-hydroxyphenyl)propionate] by I.R. and NMR spectroscopy. The hexane soluble fraction was stripped and the residue dissolved in hot carbon tetrachloride. A solid was isolated that melts at 81—84°C. This compound was the title compound.

Example 23

2-[2-(3,5-Di-t-butyl-4-hydroxyphenyl)ethyl]-4-[(hexanoyloxy)methyl]-2-oxazoline-4-methyl[3-(3,5-di-t-butyl-4-hydroxyphenyl)propionate]

In a 1 liter, three-neck, round-bottom flask equipped with a thermometer, stirrer and a Stark and Dean trap with condenser was placed 55.6 g (0.2 mole) of 3-(3,5-di-t-butyl-4-hydroxyphenyl)propionic acid, 201 g (0.1 mole) of 4,4-bis(hydroxymethyl)-2-pentyl-2-oxazoline and 200 ml of xylene. The mixture was heated to reflux and 1.8 ml of water collected. The temperature was raised to 185—190°C by removing the xylene, and the remainder of the water was collected. The mixture was cooled and hexane added. The product crystallized and one recrystallization from hexane gave the product that melted at 115—116°C.

Example 24

2-[2-(3,5-Di-t-butyl-4-hydroxyphenyl)ethyl]-4-[(dodecanoyloxy)methyl]-2-oxazoline-4-methyl [3-(3,5-di-t-butyl-4-hydroxyphenyl)propionate]

In a 1 liter, three-neck, round-bottom flask equipped with a thermometer, stirrer and a Stark and Dean trap with condenser was placed 55.6 g (0.2 mole) of 3-(3,5-di-t-butyl-4-hydroxyphenyl)propionate, 28.5 g (0.1 mole) of 4,4-bis(hydroxymethyl)-2-undecyl-2-oxazoline and 200 ml of xylene. The mixture was heated to 140°C and then to 190°C and 3.4 ml of water were collected. The reaction mixture was dissolved in hot hexane and a solid separated on cooling. One recrystallization from methanol gave the title compound melting at 122—124°C.

Example 25

2-[2-(3,5-Di-t-butyl-4-hydroxyphenyl)ethyl]-4-[(Stearoyloxy)methyl]-2-oxazoline-4-methyl [3-(3,5-di-t-butyl-4-hydroxyphenyl)propionate]

In a 1 liter, three-neck, round-bottom flask equipped with a thermometer, stirrer and a Stark and Dean trap with condenser was placed 55.6 g (0.2 mole) of 3-(3,5-di-t-butyl-4-hydroxyphenyl) propionic acid, 36.9 g (0.1 mole) of 2-heptadecyl-4,4-bis(hydroxymethyl)-2-oxazoline and 300 ml of diethylbenzene. The mixture was refluxed for 23 hours and 3.6 ml of water was collected. The diethylbenzene was removed and the residue dissolved in hot methanol and allowed to cool. The product was removed by filtration and dried. It melted at 102—104°C.

Example 26

2-[2-(3,5-Di-t-butyl-4-hydroxyphenyl)ethyl]-4-[(benzoxy)methyl]-2-oxazoline-4-methyl [3-(3,5-di-t-butyl-4-hydroxyphenyl)propionate]

In a 1 liter, three-neck, round-bottom flask equipped with a thermometer, stirrer and a Stark and Dean trap with condenser was placed 55.6 g (0.2 mole) of 3-(3,5-di-t-butyl-4-hydroxyphenyl) propionic acid, 20.7 g (0.1 mole) of 4,4-bis(hydroxymethyl)-2-phenyl-2-oxazoline and 300 ml of diethylbenzene. The

mixture was refluxed for 16 hours to remove the water. The ethylbenzene was removed and the residue dissolved in hot hexane and allowed to cool. The product was removed by filtration and recrystallized from hexane. It melted at 156—160°C.

Example 27

2,2'-Tetramethylenebis(2-oxazoline)-4,4,4',4'-tetramethyl tetrakis [3-(3,5-di-t-butyl-4-hydroxyphenyl)propionate]

In a 1 liter, three-neck, round-bottom flask equipped with a thermometer, stirrer and a Stark and Dean trap with condenser was placed 55.6 g (0.2 mole) of 3-(3,5-di-t-butyl-4-hydroxyphenyl) propionic acid, 31.6 g (0.1 mole) of 2,2'-tetramethylene bis [4, 4-bis(hydroxymethyl)-2-oxazoline] and 200 ml of xylene. The mixture was heated to reflux and 3.6 ml of water was collected. Then 55.6 g (0.2 mole) of 3-(3,5-di-t-butyl-4-hydroxyphenyl)propionic acid was added and reflux continued. As no water was then removed by distillation, the temperature was raised to 185—190°C, by removing xylene, and 3.6 ml of water was collected. The reaction mixture was dissolved in hot hexane and allowed to cool. The product separated as an oil so it was redissolved in hot hexane and allowed to cool with stirring. Again, the product separated as an oil. However, on continued stirring overnight, a solid material, melting at 85—100°C was isolated. The solid was dissolved in acetone, and was filtered and solidified from hexane as described above. It melted at 75—80°C. The product was dissolved in chloroform, passed through an alumina column and resolidified from hexane, as above. It then melted at 81—85°C.

Example 28

2,2'-Thiodiethylenebis(2-oxazoline)-4,4,4',4'-tetramethyl tetrakis [3-(3,5-di-t-butyl-4-hydroxyphenyl)propionate]

In a 1 liter, three-neck, round-bottom flask equipped with a thermometer, stirrer and a Stark and Dean trap with condenser was placed 111.6 gm (0.4 moles) of 3-(3,5-di-t-butyl-4-hydroxyphenyl) propionic acid, 34.8 gm (0.1 moles) of 2,2'-thiodiethylene bis[4,4-bis(hydroxymethyl)-2-oxazoline] and 200 ml of decalin. The mixture was heated to 195°C and the water was removed by azeotropic distillation. The decalin was stripped off and the residue was dissolved in hot hexane and cooled with stirring until the product solidified. The product melted at 79—85°C. It was dissolved in chloroform, passed through an alumina column and resolidified from hexane, as described above. It melted at 84—87°C.

Example 29

2-n-Heptadecyl-2-oxazoline-4,4-dimethyl bis[3-(3,5-di-t-butyl-4-hydroxyphenyl)propionate]

In a 1 liter, three-neck, round-bottom flask equipped with a thermometer, stirrer, dropping funnel and condenser was placed 36.9 g (0.1 mole) of 2-n-heptadecyl-4,4-bis(hydroxymethyl)-2-oxazoline, 50 ml of triethylamine and 300 ml of hexane. To this stirred slurry was added 59.3 g (0.2 mole) of 3-(3,5-di-t-butyl-4-hydroxyphenyl)propionyl chloride in hexane. The reaction mixture was refluxed for 1 hour and filtered. The solvent was stripped off. The residue was dissolved in hot hexane. The product did not crystallize so the hexane solution was chromotragraphed on alumina. The product fraction still did not crystallize. The hexane was stripped off and the product was a low melting resin.

Example 30

2-Phenyl-2-oxazoline-4,4-dimethyl bis[3-(3,5-di-t-butyl-4-hydroxyphenyl)propionate]

In a 1 liter, three-neck, round-bottom flask equipped with a thermometer, stirrer, dropping funnel and condenser was placed 41.4 g (0.2 mole) of 4,4-bis(hydroxymethyl)-2-phenyl-2-oxazoline and 250 ml of pyridine. To this stirred solution was added 118.6 g (0.4 mole) of 3-(3,5-di-t-butyl-4-hydroxyphenyl)propionyl chloride in 100 ml of toluene. While the temperature was 25—30°C. The mixture was stirred overnight and then filtered. The solvent was stripped off and the residue dissolved in 350 ml of hot toluene. More pyridine hydrochloride precipitated and it was removed by filtration. The solvent was stripped off and the residue was dissolved in hot isopropanol. The product crystallized and it was removed by filtration and dried. It melted at 123—125°C. On recrystallization from isopropanol, the product melted at 128—129°C.

Example 31

2-(4-t-Butylphenyl)-2-oxazoline-4,4-dimethyl bis[3-(3,5-di-t-butyl-4-hydroxyphenyl)propionate]

In a 1 liter, three-neck, round-bottom flask equipped with a thermometer, stirrer, dropping funnel and condenser was placed 52.6 g (0.2 mole) of 2-(4-t-butylphenyl)-4,4-bis(hydroxymethyl)-2-oxazoline and 200 ml of pyridine. To this stirred mixture was added 118.6 g (0.4 mole) of 3-(3,5-di-t-butyl-4-hydroxyphenyl) propionyl chloride dissolved in 400 ml of toluene. The reaction temperature was maintained at 20—30°C during the addition. The mixture was allowed to stir overnight. The mixture was filtered, stripped and dissolved in hot toluene. More pyridine hydrochloride separated. It was removed by filtration and the toluene was stripped off. The residue was dissolved in hot isopropanol and allowed to cool. The solid that separated was removed by filtration and dried. The solid melted at 105—110°C. The filtrate was stripped and dissolved in hot hexane. On cooling, a solid was isolated which melted at 104—108°C. Recrystallization from hexane gave a pure product that melted at 118—119°C.

Example 32

2-(4-Nitrophenyl)-2-oxazoline-4,4-dimethyl bis[3-(3,5-di-t-butyl-4-hydroxyphenyl)propionate]

In a 1 liter, three-neck, round-bottom flask equipped with a thermometer, stirrer, dropping funnel and condenser was placed 50.4 g (0.2 mole) of 4,4-bis(hydroxymethyl)-2-(4-nitrophenyl)-2-oxazoline and 200 ml of pyridine. To this stirred slurry was added 118.6 g (0.4 mole) of 3-(3,5-di-t-butyl-4-hydroxyphenyl)propionyl chloride dissolved in 400 ml of toluene. The reaction temperature was maintained at 20—30°C during the addition. The mixture was stirred overnight and then filtered and stripped. The residue was dissolved in hot toluene and filtered to remove pyridine hydrochloride. The toluene was stripped off and the residue dissolved in hot isopropanol. The product crystallized and was removed by filtration. After one recrystallization from isopropanol, the product melted at 138—140°C.

Example 33

Bis(2-[2-(3,5-di-t-butyl-4-hydroxyphenyl)ethyl]-4-methyl-2-oxazoline-4-methyl) oxalate

In a 1 liter, three-neck, round-bottom flask equipped with a thermometer, stirrer, dropping funnel and condenser was placed 67.0 g (0.193 mole) of 4-hydroxymethyl-4-methyl-2-[2-(3,5-di-t-butyl-4-hydroxyphenyl)ethyl]-2-oxazoline, 300 ml of xylene and 50 ml of triethylamine. To this stirred slurry was added 12.5 g (0.097 mole) of 98% oxalyl chloride. The mixture was filtered hot and the solvent stripped off. The residue was dissolved in hot toluene and allowed to cool. The product crystallized and was removed by filtration and dried. The product melted at 188—190°C.

Example 34

Bis(2-[2-(3,5-di-t-butyl-4-hydroxyphenyl)ethyl]-4-ethyl-2-oxazoline-4-methyl) oxalate

In a 1 liter, three-neck, round-bottom flask equipped with a thermometer, stirrer, dropping funnel and condenser was placed 69.7 (0.193 mole) of 4-ethyl-4-hydroxymethyl-2-[2-(3,5-di-t-butyl-4-hydroxyphenyl)-ethyl]-2-oxazoline, 300 ml of xylene and 50 ml of triethylamine. To this, stirred slurry was added 12.5 g (0.097 mole) of oxalyl chloride. The mixture was stirred for 3 hours and then heated to 90°C. The mixture was filtered hot and the solvent stripped off. The residue was dissolved in hot toluene and allowed to cool. The product crystallized and was removed by filtration and dried. The product melted at 157—160°C.

Example 35

Stabilization of Polypropylene

The antioxidant (0.1 parts by weight) was blended into Profax 6501 (trademark of Hercules, Inc. for polypropylene) resin (100 parts by weight) on a mill at 350°F (177°C). This stabilized polypropylene was pressed into 75 mil sheets and then buttons punched from these sheets. The same procedure was followed in order to prepare buttons containing 0.1 parts of antioxidant and 0.25 parts of distearyl-thiodipropionate. Three buttons of each sample of stabilized polymer were aged at 149°C (300°F) in a circulating air oven and the days to embrittlement recorded. The break point is defined as the first sight of embrittlement or crumbling in two of the three buttons.

| Compound | Days to Break | |
|---|---|---|
| | Alone | With DSTDP |
| Blank | 1 | 4 |
| 2-(3,5-Di-t-butyl-4-hydroxybenzyl)-2-(4,4-dimethyl-2-oxazoline)trimethylenebis-[3-(3,5-di-t-butyl-4-hydroxy phenyl)propionate] | 19 | 118 |
| 2-[2-(3,5-Di-t-butyl-4-hydroxyphenyl)-ethyl]-4-ethyl-2-oxazoline-4-methyl [3-(3,5-di-t-butyl-4-hydroxyphenyl)-propionate] | 24 | 59 |
| 2-[2-(3,5-Di-t-butyl-4-hydroxyphenyl)-ethyl]-2-oxazoline-4,4-dimethyl bis[3-(3,5-di-t-butyl-4-hydroxyphenyl)-propionate] | 34 | 90 |
| 2-[2-(3,5-Di-t-butyl-4-hydroxyphenyl)-ethyl]-4-hydroxymethyl-2-oxazoline-4-methyl [3-(3,5-di-t-butyl-4-hydroxy-phenyl)propionate] | 32 | 50 |
| Bis(2-[2-(3,5-di-t-butyl-4-hydroxy-phenyl)ethyl]-4-methyl-2-oxazoline-4-methyl) oxalate | 25 | 19 |
| Bis(2-[2-(3,5-di-t-butyl-4-hydroxy-phenyl)ethyl]-4-ethyl-2-oxazoline-4-methyl)oxalate | 22 | 25 |
| 2-[2-(3,5-Di-t-butyl-4-hydroxyphenyl)-ethyl]-4-[(acetoxy)methyl]-2-oxazoline-4-methyl [3-(3,5-di-t-butyl-4-hydroxy-phenyl)propionate] | 39 | 81 |
| 2-[2-(3,5-Di-t-butyl-4-hydroxyphenyl)-ethyl]-4-[(hexanoyloxy)methyl]-2-oxazoline-4-methyl[3-(3,5-di-t-butyl-4-hydroxyphenyl)propionate] | 28 | 111 |
| 2-[2-(3,5-Di-t-butyl-4-hydroxyphenyl)-ethyl]-4-[(dodecanoyloxy)methyl]-2-oxazoline-4-methyl [3-(3,5-di-t-butyl-4-hydroxyphenyl)propionate] | 37 | 104 |
| 2-[2-(3,5-Di-t-butyl-4-hydroxyphenyl)-ethyl]-4-[(stearoyloxy)methyl]-2-oxazoline-4-methyl [3-(3,5-di-t-butyl-4-hydroxyphenyl)propionate] | 34 | 93 |
| 2-[2-(3,5-Di-t-butyl-4-hydroxyphenyl)-ethyl]-4-[(benzoxy)methyl]-2-oxazoline-4-methyl [3-(3,5-di-t-butyl-4-hydroxy-phenyl)propionate] | 39 | 68 |
| 2-Methyl-2-oxazoline-4,4-dimethyl bis-[3-(3,5-di-t-butyl-4-hydroxyphenyl) propionate] | 27 | 68 |

| Compound | Days to Break Alone | Days to Break With DSTDP |
|---|---|---|
| 2-n-Heptadecyl-2-oxazoline-4,4-dimethyl bis[3-(3,5-di-t-butyl-4-hydroxyphenyl) propionate] | 28 | 44 |
| 2,2'-Tetramethylene bis(2-oxazoline)-4 4,4',4'-tetramethyl tetrakis[3-(3,5-di-t-butyl-4-hydroxyphenyl)propionate] | 27 | 98 |
| 2,2'-Thiodiethylene bis(2-oxazoline)-4,4,4',4'-tetramethyl tetrakis [3-(3,5-di-t-butyl-4-hydroxyphenyl)-propionate] | 41 | 91 |
| 2-Phenyl-2-oxazoline-4,4-dimethyl bis[3-(3,5-di-t-butyl-4-hydroxy-phenyl)propionate] | 34 | 133 |
| 2-(4-t-Butylphenyl)-2-oxazoline-4,4-dimethyl bis[3-(3,5-di-t-butyl-4-hydroxyphenyl)propionate] | 27 | 69 |
| 2-(4-Nitrophenyl)-2-oxazoline-4,4-dimethyl bis[3-(3,5-di-t-butyl-4-hydroxyphenyl)propionate] | 39 | 77 |

Example 36

This example shows the usefulness of the compounds of the present invention in stabilizing an ethylene/propylene terpolymer and their superiority to the compounds of the prior art.

The stabilizers (0.165 g) were dissolved in a rubber cement containing 110 g EPDM (ethylene/propylene 5-ethylidene-2-norbornene having an ethylene/propylene weight ratio of 67/33, an iodine number of 10 and a Mooney viscosity (ML—4 at 20°C) of 57) in 2,000 g hexane. The hexane was removed by slowly adding the cement to boiling water. The solids were dried on the mill for 5 minutes at 135—149°C (275—300°F).

The time necessary for a 1 g sample to absorb 20 cc of oxygen at 150°C was then determined.

| Additive | $T_{20}$ (Minutes |
|---|---|
| Blank | 27 |
| Compound of Example 5 | 420+ |
| Compound of Example 8 | 420+ |

Example 37

This example shows the usefulness of the compounds of the present invention in stabilizing a polypropylene/ethylene-propylene terpolymer blend.

The compound was milled into a polypropylene/ethylene-propylene terpolymer blend at a concentration of 0.3 parts per hundred. Seventy-five mil plaques were compression molded and buttons were punched from these plaques. The buttons were exposed to a force air oven at 149°C (300°F) and the time for three of five buttons to embrittle was noted.

| Addition | Hours to Embrittlement |
|---|---|
| None | 80 |
| Compound of Example 8 | 214 |

**Claims for the Contracting States: BE CH DE FR GB IT LI NL**

1. A substituted oxazoline characterised by having the structural formula

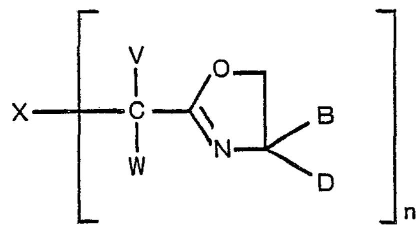

wherein B and D are the same or different and are methyl, ethyl, $—CH_2OH$ or $CH_2OOCY$, wherein Y is $C_1—C_{17}$ alkyl, phenyl or A; D may also be $—CH_2OOCZ$, wherein Z is a single bond connecting two groups of the said structural formula; A is 2-(3,5-di-t-butyl-4-hydroxyphenyl)ethyl; X is $C_1—C_{17}$ alkyl, phenyl, t-butylphenyl, nitrophenyl, hydroxyphenyl, chlorophenyl, (3,5-di-t-butyl-4-hydroxyphenyl)methyl or a divalent group selected from $C_1—C_2$ alkylene or thiodimethylene; V and W may be the same or different and are hydrogen, $—CH_2OH$ or $—CH_2OOCA$, wherein A has the meaning above; n is equal to 1 or 2; provided that X is not a divalent group when D is $—CH_2OOCZ$ and further provided that at least one of B, D, V, W and A contains a (3,5-di-t-butyl-4-hydroxyphenyl) moiety.

2. A stabilised composition comprising an organic material normally subjected to deterioration by the influence of oxygen, light or heat characterised by said material containing from 0.001 to 10 parts per 100 parts of said material a substituted oxazoline as described in claim 1.

**Claims for the Contracting State: AT**

1. A method for preparing substituted oxazolines having the structural formula

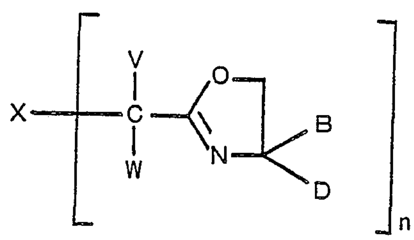

(I)

wherein B and D are the same or different and are methyl, ethyl, $—CH_2OH$ or $—CH_2OOCY$, wherein Y is $C_1—C_{17}$ alkyl, phenyl or A; D may also be $—CH_2OOCZ$, wherein Z is a single bond connecting two groups of the said structural formula; A is 2-(3,5-di-t-butyl-4-hydroxy-phenyl)ethyl; X is $C_1—C_{17}$ alkyl, phenyl, t-butylphenyl, nitrophenyl, hydroxyphenyl, chlorophenyl, (3,5-di-t-butyl-4-hydroxyphenyl)methyl or a divalent group selected from $C_1—C_2$ alkylene or thiodimethylene; V and W may be the same or different and are hydrogen, $—CH_2OH$ or $—CH_2OOCA$, wherein A has the meaning as above; n is equal to 1 or 2; provided that X is not a divalent group when D is $—CH_2OOCZ$ and further provided that at least one of B, D, V, W and A contains a (3,5-di-t-butyl-4-hydroxyphenyl) moiety, characterized in that a carboxylic acid having the structural formula

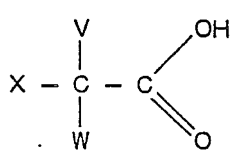

(II)

wherein X, V and W are defined as above, or an halogenide or an ester of said carboxylic acid is reacted at a temperature of 90—220°C with a beta-amino alcohol having the structural formula

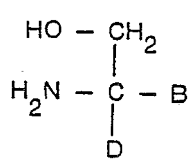

(III)

wherein B and D are defined as above, and in that the oxazoline thus obtained provided it has at least one hydroxy group is optionally esterified.

2. A method according to claim 1, characterized in that the reaction between the carboxylic acid or its halogenide or ester and the beta-amino alcohol is carried out in a solvent.

3. A method according to claim 1 or claim 2, characterized in that formic, acetic, propionic, butyric, hexanoic, decanoic, lauric, stearic, cyclohexanecarboxylic, benzoic, 4-nitrobenzoic, 4-t-butylbenzoic, 2-chlorobenzoic, salicylic, phenylacetic, adipic, sebacic, azaleic, terephthalic, toluic, thiodipropionic or 3-(3,5-di-t-butyl-4-hydroxyphenyl)propionic acid is reacted with the beta-amino alcohol (III).

4. A method according to any of claims 1 and 2, characterized in that methyl formate, ethyl acetate, ethyl propionate, methyl laurate, methyl stearate, methyl benzoate, methyl salicylate, dimethyl adipate, dimethyl terephthalate, dimethyl thiodipropionate or methyl 3-(3,5-di-t-butyl-4-hydroxyphenyl)propionate is reacted with the beta-amino alcohol (III).

5. A method according to any of claims 1 to 4, characterized in that the carboxylic acid (II) is reacted with ethanolamine, 2-amino-1-propanol, 2-amino-2-methyl-1-propanol, 2-amino-2-methylpropanediol-1,3, 2-amino-ethylpropanediol-1,3, 2-amino-1-butanol and tris(hydroxymethyl)-aminomethane or 1-isoleucinol.

6. A method according to any of claims 1 to 5, characterized in that the oxazoline (I) obtained provided it has at least one unreacted hydroxyl group, is esterified with formic, acetic, propionic, butyric, hexanoic, decanoic, lauric, stearic, cyclohexanecarboxylic, benzoic, 4-nitrobenzoic, 4-t-butylbenzoic, 2-chlorobenzoic, salicyclic, phenylacetic, oxalic, succinic, maleic, malonic, adipic, sebacic, azelaic, phthalic, terephthalic, toluic, thiodipropionic or 3-(3,5-di-t-butyl-4-hydroxyphenyl)propionic acid or with formoyl, acetyl, propionoyl, hexanoyl, dodecanoyl, stearoyl, benzoyl, 4-nitrobenzoyl, 2-chlorobenzoyl, oxalyl, succinoyl, adipoyl, sebacyl, phthaloyl, terephthaloyl, thiodipropionyl or 3-(3,5-di-t-butyl-4-hydroxyphenyl)propionyl chloride.

7. A stabilised composition comprising an organic material normally subject to deterioration by the influence of oxygen, light or heat characterized by said material containing from 0,001 to 10 parts per 100 parts of said material a substituted oxazoline having the structural formula

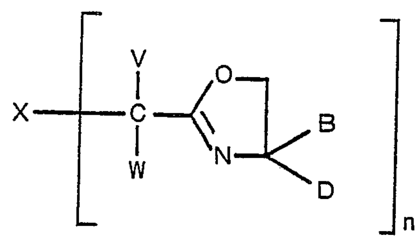

(I)

wherein B and D are the same or different and are methyl, ethyl, $—CH_2OH$ or $CH_2OOCY$, wherein Y is $C_1—C_{17}$ alkyl, phenyl or A; D may also be $—CH_2OOCZ$, wherein Z is a single bond connecting two groups of the said structural formula; A is 2-(3,5-di-t-butyl-4-hydroxyphenyl)ethyl; X is $C_1—C_{17}$ alkyl, phenyl, t-butylphenyl, nitrophenyl, hydroxyphenyl, chlorophenyl, (3,5-di-t-butyl-4-hydroxyphenyl)methyl or a divalent group selected from $C_1—C_2$ alkylene or thiodimethylene; V and W may be the same or different and are hydrogen, $—CH_2OH$ or $—CH_2OOCA$, wherein A has the meaning as above; n is equal to 1 or 2; provided that X is not a divalent group when D is $—CH_2OOCZ$ and further provided that at least one of B, D, V, W and A contains a (3,5-di-t-butyl-4-hydroxyphenyl) moiety.

**Revendications pour les Etats contractants: BE CH DE FR GB IT LI NL**

1. Oxazoline substituée caractérisée en ce qu'elle a la formule de structure

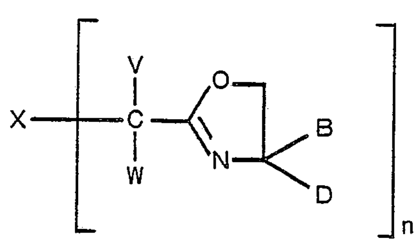

dans laquelle B et D sont identiques ou différents et sont méthyle, éthyle, $—CH_2OH$ ou $CH_2OOCY$, où Y est un alcoyle $C_1—C_{17}$, du phényle ou A; D peut également être $—CH_2OOCZ$ où Z est une simple liaison reliant deux groupes de ladite formule de structure; A est 2-(3,5-di-t-butyl-4-hydroxyphényl)éthyle, X est un alcoyle $C_1—C_{17}$, du phényle, du t-butylphényle, du nitrophényle, de l'hydroxyphényle, du chlorophényle, du (3,5-di-t-butyl-4-hydroxyphényl)méthyle ou un groupe divalent choisi parmi un alcoylène $C_1—C_2$ ou du

thiodiméthylène; V et W peuvent être identiques ou différents et sont de l'hydrogène, $—CH_2OH$ ou $—CH_2OOCA$, où A a la signification ci-dessus; n est égal à 1 ou 2; à condition que X ne soit pas un groupe divalent quand D est $—CH_2OOCZ$ et à condition de plus qu'au moins l'un de B, D, V, W et A contienne un fragment (3,5-di-t-butyl-4-hydroxyphényle).

2. Composition stabilisée comprenant une matière organique normalement sujette à une détérioration par l'influence de l'oxygène, de la lumière ou de la chaleur, caractérisée en ce que ladite matière contient de 0,001 à 10 parties pour 100 parties de ladite matière d'une oxazoline substituée selon la revendication 1.

**Revendications pour l'Etat contractant: AT**

1. Procédé de préparation d'oxaz:olines substituées ayant la formule de structure

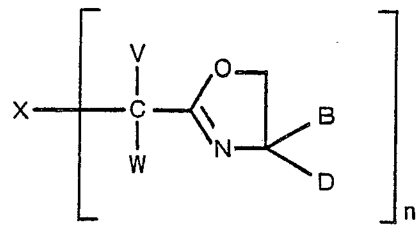

dans laquelle B et D sont identiques ou différents et sont méthyle, éthyle, $—CH_2OH$ ou $CH_2OOCY$, où Y est un alcoyle $C_1—C_{17}$, du phényle ou A; D peut également être $—CH_2OOCZ$ où Z est une simple liaison reliant deux groupes de ladite formule de structure; A est 2-(3,5-di-t-butyl-4-hydroxyphényl)éthyle, X est un alcoyle $C_1—C_{17}$, du phényle, du t-butylphényle, du nitrophényle, de l'hydroxyphényle, du chlorophényle, du (3,5-di-t-butyl-4-hydroxyphényl)méthyle ou un groupe divalent choisi parmi un alcoylène $C_1—C_2$ ou du thiodiméthylène; V et W peuvent être identiques ou différents et sont de l'hydrogène, $—CH_2OH$ ou $—CH_2OOCA$, où A a la signification ci-dessus; n est égal à 1 ou 2; à condition que X ne soit pas un groupe divalent quand D est $—CH_2OOCZ$ et à condition de plus qu'au moins l'un de B, D, V, W et A contienne un fragment (3,5-di-t-butyl-4-hydroxyphényle), caractérisé en ce que l' on fait réagir un acide carboxylique ayant la formule de structure

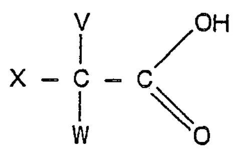

(II)

où X, V et W sont définis ci-dessus, ou un halogénure ou un ester dudit acide carboxylique, à une température de 90 à 220°C avec un béta-amino alcool ayant la formula de structure

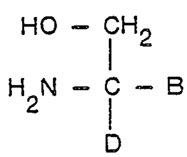

(III)

où B et D sont tels que définis ci-dessus et en ce que l'oxazoline ainsi obtenue, à condition qu'elle ait au moins un groupe hydroxy, est éventuellement esterifiée.

2. Procédé selon la revendication 1 caractérisé en ce que la réaction entre l'acide carboxylique ou son halogénure ou ester et le béta-amino alcool est effectuée dans un solvant.

3. Procédé selon l'une quelconque des revendications 1 ou 2 caractérisé en ce que l'on fait réagir de l'acide formique, acétique, propionique, butyrique, hexanoique, décanoique, laurique, stéarique, cyclohexanecarboxylique, benzoique, 4-nitrobenzoique, 4-t-butylbenzoique, 2-chlorobenzoique, salicylique, phénylacétique, adipique, sébacique, azaléique, téréphtalique, toluique, thiodipropionique ou 3-(3,5-di-t-butyl-4-hydroxyphényl) propionique avec le béta-amino alcool (III).

4. Procédé selon l'une quelconque des revendications 1 ou 2 caractérisé en ce que l'on fait réagir le formiate de méthyle, l'acétate d'éthyle, le propionate d'éthyle, le laurate de méthyle, le stéréate de méthyle, le benzoate de méthyle, le salicylate de méthyle, l'adipate de diméthyle, le téréphtalate de diméthyle, le thiodipropionate de diméthyle ou le 3-(3,5-di-t-butyl-4-hydroxyphényl) propionate de méthyle avec le béta-amino alcool (III).

5. Procédé selon l'une quelconque des revendications 1 à 4 caractérisé en ce que l'on fait réagir l'acide carboxylique (II) avec de l'éthanolamine, du 2-amino-1-propanol, du 2-amino-2-méthyl-1-propanol, du 2-amino-2-méthylpropanediol-1,3, du 2-amino-éthylpropanediol-1,3, du 2-amino-1-butanol et du tris (hydroxyméthyl)-aminoéthane ou du 1-isoleucinol.

6. Procédé selon l'une quelconque des revendications 1 à 5 caractérisé en ce que l'oxazoline (I) obtenue à condition qu'elle ait au moins un groupe hydroxyle n'ayant pas réagi, est estérifiée avec de l'acide formique, acétique, propionique, butyrique, hexanoique, décanoique, laurique, stéarique, cyclohexanecarboxylique, benzoique, 4-nitrobenzoique, 4-t-butylbenzoique, 2-chlorobenzoique, salicylique, phénylacétique, oxalique, succinique, maléique, malonique, adipique, sébacique azélaique, phtalique, téréphtalique, toluique, thiodipropionique ou 3-(3,5-di-t-butyl-4-hydroxyphényl) propionique ou avec du chlorure de formoyle, d'acétyle, de propionoyle, d'hexanoyle, de dodécanoyle, de stéaroyle, de benzo le, de 4-nitrobenzoyle, de 2-chlorobenzoyle, d'oxalyle, de succinoyle, d'adipoyle, de sébacyle, de phtaloyle, de téréphtaloyle, de tiodipropionyle, ou de 3-(3,5-di-t-butyl-4-hydroxyphényl) propionyle.

7. Composition stabalisée comprenant une matière organique normalement sujette à une détérioration par l'influence de l'oxygène, de la lumière ou de la chaleur caractérisée en ce que ladite matière contient de 0,001 à 10 parties pour 100 parties de ladite matière d'une oxazoline substituée ayant la formule de structure

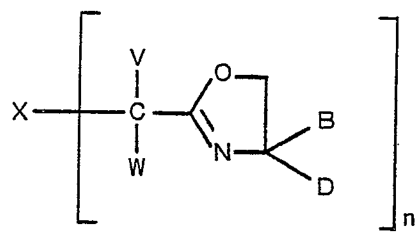

(I)

dans laquelle B et D sont identiques ou différents et sont méthyle, éthyle, $—CH_2OH$ ou $CH_2OOCY$, où Y est un alcoyle $C_1—C_{17}$, du phényle ou A; D peut également être $—CH_2OOCZ$, où Z est une simple liaison reliant deux groupes de ladite formule de structure; A est 2-(3,5-di-t-butyl-4-hydroxyphényl) éthyle, X est un alcoyle $C_1—C_{17}$, du phényle, du t-butylphényle, du nitrophényle, de l'hydroxyphényle, du chlorophényle, du (3,5-di-t-butyl-4-hydroxyphényl) méthyle ou un groupe divalent choisi parmi un alcoylène $C_1—C_2$ ou du thiodiméthylène; V et W peuvent être identiques ou différents et sont de l'hydrogène, $—CH_2OH$ ou $—CH_2OOCA$, où A a la signification ci-dessus; n est égal à 1 ou 2; à condition que X ne soit pas un groupe divalent lorsque D est $—CH_2OOCZ$ et de plus à condition qu'au moins l'un de B, D, V, W et A contienne un fragment (3,5-di-t-butyl-4-hydroxyphényle).

**Patentansprüche für die Vertragsstaaten: BE CH DE FR GB IT LI NL**

1. Substituiertes Oxazolin, gekennzeichnet durch die Strukturformel:

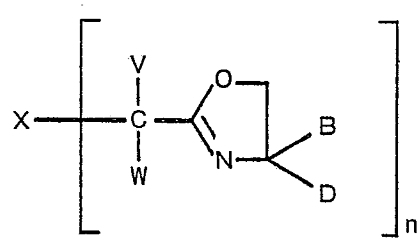

worin B und D gleich oder verschieden sind und jeweils einen Methylrest, einen Ethylrest, $—CH_2OH$ oder $—CH_2OOCY$, worin Y ein $C_1$- bis $C_{17}$-Alkylrest, ein Phenylrest oder A ist, bedeuten; D auch $—CH_2OOCZ$, worin Z eine Einfachbindung ist, die zwei Gruppen der vorstehenden Strukturformel verbindet, sein kann; A ein 2-(3,5-Di-t-butyl-4-hydroxyphenyl)-ethylrest ist; X ein $C_1$- bis $C_{17}$-Alkyl-, Phenyl-, t-Butylphenyl-, Nitrophenyl-, Hydroxyphenyl-, Chlorophenyl- oder (3,5-Di-t-butyl-4-hydroxyphenyl)-methylrest oder eine aus $C_1$- bis $C_2$-Alkylenresten oder dem Thiodimethylenrest ausgewählte zweiwertige Gruppe ist; V und W gleich oder verschieden sein können und Wasserstoff, $—CH_2OH$ oder $—CH_2OOCA$, worin A die vorstehende Bedeutung hat, bedeuten und n 1 oder 2 ist, wobei vorausgesetzt ist, daß X keine zweiwertige Gruppe ist, wenn D $—CH_2OOCZ$ ist, und wobei des weiteren vorausgesetzt ist, daß mindestens einer der Reste B, D, V, W und A eine (3,5-Di-t-butyl-4-hydroxyphenyl)-Komponente enthält.

2. Stabilisierte Masse, die ein organisches Material enthält, das normalerweise für eine Zersetzung durch die Einwirkung von Sauerstoff, Licht oder Wärme anfällig ist, dadurch gekenn--zeichnet, daß das Material 0,001 bis 10 Teile eines substituierten Oxazolins, wie es in Anspruch 1 beschrieben ist, pro 100 Teile des Materials enthält.

**Patentansprüche für den Vertragsstaat: AT**

1. Verfahren zum Herstellen substituierter Oxazoline mit der Strukturformel

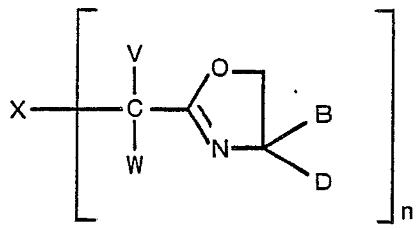

(I)

worin B und D gleich oder verschieden sind und Methyl, Äthyl, $—CH_2OH$ oder $—CH_2OOCY$ sind, worin Y $C_1—C_{17}$ Alkyl, Phenyl oder A ist; D kann auch $—CH_2OOCZ$ sein, worin Z eine Einfachbindung ist, welche zwei Gruppen der Strukturformel verbindet; A ist 2-(3,5-Di-t-butyl-4-hydroxyphenyl)äthyl; X ein $C_1—C_{17}$ Alkyl, Phenyl, t-Butylphenyl, Nitrophenyl, Hydroxyphenyl, Chlorophenyl, (3,5-Di-t-butyl-4-hydroxyphenyl)-methyl oder eine divalente Gruppe, ausgewählt unter $C_1—C_2$ Alkylen oder Thiodimethylen; V und W können gleich oder verschieden sein und sind Wasserstoff, $—CH_2OH$ oder $—CH_2OOCA$, worin A die oben genannte Bedeutung hat; n ist gleich 1 oder 2 mit der Maßgabe, daß X keine divalente Gruppe ist, wenn D $—CH_2OOCZ$ ist und weiters mit der Maßgabe, daß wenigstens eines von B, D, V, W und A eine (3,5-Di-t-butyl-4-hydroxyphenyl)-Gruppierung enthält, dadurch gekennzeichnet, daß eine Carbonsäure mit der Strukturformel

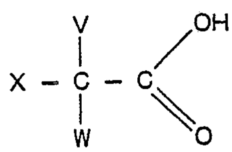

(II)

worin X, V und W wie oben definiert sind oder ein Halogenid oder ein Ester der Carbonsäure bei einer Temperatur von 90—220°C mit einem Beta-Aminoalkohol mit der Strukturformel

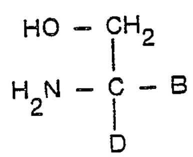

(III)

umgesetzt wird, worin B und D wie oben definiert sind, und daß das so erhaltene Oxazolin gegebenenfalls verestert wird, vorausgesetzt, daß es zumindest eine Hydroxygruppe enthält.

2. Verfahren nach Anspruch 1, dadurch gekennzeichnet, daß die Reaktion zwischen der Carbonsäure oder ihrem Halogenid oder Ester und dem Beta-Aminoalkohol in einem Lösungsmittel ausgeführt wird.

3. Verfahren nach Anspruch 1 oder 2, dadurch gekennzeichnet, daß Ameisensäure, Essigsäure, Propionsäure, Buttersäure, Hexansäure, Decansäure, Laurinsäure, Stearinsäure, Cyclohexancarbonsäure, Benzoesäure, 4-Nitrobenzoesäure, 4-t-Butylbenzoesäure, 4-Chlorobenzoesäure, Salicylsäure, Phenylessigsäure, Adipinsäure, Sebacinsäure, Azelainsäure, Terephthalsäure, Toluolsäure, Thiodipropionsäure oder 3-(3,5-Di-t-butyl-4-hydroxyphenyl)propionsäure, mit dem Beta-Aminoalkohol (III) umgesetzt wird.

4. Verfahren nach Anspruch 1 oder 2, dadurch gekennzeichnet, daß Methylformiat, Äthylacetat, Äthylpropionat, Methyllaurat, Methylstearat, Methylbenzoat, Methylsalicylat, Dimethyladipat, Dimethylterephthalat, Dimethylthiodipropionat oder Methyl-3-(3,5-di-t-butyl-4-hydroxyphenyl)propionat mit dem Beta-Aminoalkohol (III) umgesetzt wird.

5. Verfahren nach einem der Ansprüche 1 bis 4, dadurch gekennzeichnet, daß die Carbonsäure (II) mit Äthanolamin, 2-Amino-1-propanol, 2-Amino-2-methyl-1-propanol, 2-Amino-2-methylpropandiol-1,3, 2-Amino-äthylpropandiol-1,3, 2-Amino-1-butanol und Tris(hydroxymethyl)-aminomethan oder 1-Isoleucinol umgesetzt wird.

6. Verfahren nach einem der Ansprüche 1 bis 5, dadurch gekennzeichnet, daß das erhaltene Oxazolin (I), vorausgesetzt, daß es wenigstens eine unreagierte Hydroxylgruppe besitzt, mit Ameisensäure, Essigsäure, Propionsäure, Buttersäure, Hexansäure, Decansäure, Laurinsäure, Stearinsäure, Cyclohexancarbonsäure, Benzoesäure, 4-Nitrobenzoesäure, 4-t-Butylbenzoesäure, 4-Chlorobenzoesäure, Salicylsäure, Phenylessigsäure, Oxalsäure, Bernsteinsäure, Maleinsäure, Malonsäure, Adipinsäure, Sebacinsäure, Azelainsäure, Phthalsäure, Terephthalsäure, Toluolsäure, Thiodipropionsäure oder 3-(3,5-Di-t-butyl-4-hydroxyphenyl)propionsäure oder mit Formoylchlorid, Acetylchlorid, Propionylchlorid, Hexanoylchlorid, Dodecanoylchlorid, Stearylchlorid, Benzoylchlorid, 4-Nitrobenzoylchlorid, 2-Chlorobenzoylchlorid, Oxalylchlorid,

Succinoylchlorid, Adipoylchlorid, Sebacylchlorid, Phthaloylchlorid, Terephthaloylchlorid, Thiodipropionylchlorid oder 3-(3,5-Di-t-butyl-4-hydroxyphenyl)propionylchlorid verestert wird.

7. Stabilisierte Zusammensetzung, enthaltend einen organischen Stoff, der für gewöhnlich Zerstörung durch den Einfluß von Sauerstoff, Licht oder Hitze unterliegt, dadurch gekennzeichnet, daß der Stoff 0,001 bis 10 Teile je 100 Teile des Stoffes eines substituierten Oxazolins der Strukturformel

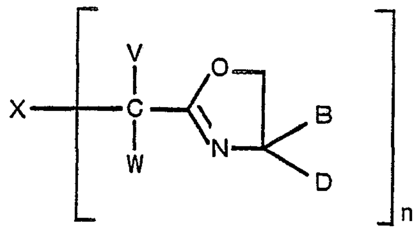

(I)

worin B und D gleich oder verschieden sind und Methyl, Äthyl, $—CH_2OH$ oder $—CH_2OOCY$ sind, worin Y $C_1—C_{17}$ Alkyl, Phenyl oder A ist; D kann auch $—CH_2OOCZ$ sein, worin Z eine Einfachbindung ist, welche zwei Gruppen der Strukturformel verbindet; A ist 2-(3,5-Di-t-butyl-4-hydroxyphenyl)äthyl; X ist $C_1—C_{17}$ Alkyl, Phenyl, t-Butylphenyl, Nitrophenyl, Hydroxyphenyl, Chlorophenyl, (3,5-Di-t-butyl-4-hydroxyphenyl)-methyl oder eine divalente Gruppe, ausgewählt unter $C_1—C_2$ Alkylen oder Thiodimethylen; V und W können gleich oder verschieden sein und sind Wasserstoff, $—CH_2OH$ oder $—CH_2OOCA$, worin A die oben genannte Bedeutung hat; n ist gleich 1 oder 2 mit der Maßgabe, daß X keine divalente Gruppe ist, wenn D $—CH_2OOCZ$ ist und weiters mit der Maßgabe, daß wenigstens eines von B, D, V, W und A eine (3,5-Di-t-butyl-4-hydroxyphenyl)-Gruppierung enthält.